# Europäisches Patentamt

# European Patent Office

# Office européen des brevets

(11) Veröffentlichungsnummer: **0 172 422**
A2

(12) **EUROPÄISCHE PATENTANMELDUNG**

(21) Anmeldenummer: 85109098.5

(22) Anmeldetag: 20.07.85

(51) Int. Cl.⁴: **A 61 K 9/52**
A 61 K 9/50, A 61 K 9/22
A 61 K 37/02

(30) Priorität: 01.08.84 DE 3428372

(43) Veröffentlichungstag der Anmeldung:
26.02.86 Patentblatt 86/9

(84) Benannte Vertragsstaaten:
AT BE CH DE FR GB IT LI LU NL SE

(71) Anmelder: HOECHST AKTIENGESELLSCHAFT
Postfach 80 03 20
D-6230 Frankfurt am Main 80(DE)

(72) Erfinder: Sandow, Jürgen Kurt, Dr.
Am Haideplacken 22
D-6240 Königstein/Taunus(DE)

(72) Erfinder: Seidel, Heinz-Rüdiger
Im Kirschenfeld 15
D-6370 Oberursel(DE)

(54) Mikrokapseln von regulatorischen Peptiden mit kontrollierter Freisetzung, Verfahren zu ihrer Herstellung und Injektionszubereitungen.

(57) Mikrokapseln von regulatorischen Peptiden mit kontrollierter Wirstoff-Freisetzung enthaltend Poly-D(-)-3-hydroxybuttersäure als Trägerstoff, Verfahren zu ihrer Herstellung sowie Injektionszubereitungen enthaltend diese Mikrokapseln werden beschrieben.

EP 0 172 422 A2

HOECHST AKTIENGESELLSCHAFT     HOE 84/F 174      Dr.D/mü

Mikrokapseln von regulatorischen Peptiden mit kontrollierter Freisetzung, Verfahren zu ihrer Herstellung
und Injektionszubereitungen

Die Erfindung betrifft Mikrokapseln von regulatorischen
Peptiden mit kontrollierter Wirkstoff-Freigabe, welche
als Trägerstoff die biologisch abbaubare Poly-D(-)-3-
hydroxybuttersäure enthalten, Verfahren zur Herstellung
dieser Mikrokapseln und Injektionszubereitungen enthaltend
diese Mikrokapseln.

Wenngleich die perorale Applikation von Arzneistoffen
in der Regel bevorzugt ist, ist sie für Peptide in vielen
Fällen nicht geeignet, da diese im Magen-Darm-Trakt enzymatisch abgebaut werden, und man daher damit rechnen muß,
daß keine therapeutisch wirksamen Blutspiegel erreicht
werden. Hier bietet sich die parenterale Applikation an.
Zur Langzeitbehandlung mit Peptiden werden Implantate
mit kontrollierter Freisetzung beschrieben. Entsprechend
den Angaben in der Literatur können als Trägermaterial
für solche Implanate verschiedene Polymere verwendet werden, wie z.B. Silicon-Elastomere und Ethylen-Vinylacetat-
Copolymere. Es können auch biologisch abbaubare Polymere
wie Polymilchsäure und Polyglykolsäure und ihre Copolymeren eingesetzt werden. Implanate aus diesem Material
müssen nicht operativ entfernt werden, da sie im Körper
zu den physiologischen Monomeren Milchsäure bzw. Glykolsäure abgebaut werdne (vgl. Europäische Patentanmeldung
mit der Veröffentlichungsnummer 0 058 481).

Implantate haben den Nachteil, daß sie auf chirurgischem
Weg eingesetzt werden müssen. Die Behandlung läßt sich
einfacher gestalten, wenn man die Peptide zu Mikrokapseln
verarbeitet und diese nach Suspension in einem physiologisch verträglichen Medium injiziert. Allerdings ist es
unabdingbar, daß biologisch abbaubare Trägerstoffe zur
Mikroverkapselung eingesetzt werden, da die Mikrokapseln wegen ihrer geringen Größe (von etwa maximal 200 µm) kaum wieder nach Injektion entfernt werden können.

Es ist auch schon beschrieben worden, daß man Peptide zu Mikrokapseln verarbeiten kann. Z.B. wird in der Europäische Patentanmeldung mit der Veröffentlichungsnummer 0 052 510 die Mikroverkapselung von LH-RH und Analoga mit Copolymeren aus Milch- und Glykolsäure nach dem Verfahren der Koazervation beschrieben.

Die Herstellung der Polymeren von Milchsäure und Glykolsäure und ihrer Copolymeren ist aufwenig. Insbesondere ist es nicht einfach, ihre Eigenschaften zu reproduzieren. Ein weiterer Nachteil dieser Polymeren besteht in möglichen Katalysatorrückständen.

Es wurde nun gefunden, daß sich Poly-D(-)-3-hydroxybuttersäure als biologisch abbaubarer Trägerstoff für peptidhaltige Mikrokapseln eignet.

Die Erfindung betrifft daher Mikrokapseln von regulatorischen Peptiden mit kontrollierter Wirkstoff-Freisetzung enthaltend Poly-D(-)-3-hydroxybuttersäure als biologisch abbaubaren Trägerstoff.

In den vorstehenden und folgenden Ausführungen steht "Peptide" für regulatorische Peptide und deren Analoga sowie deren physiologisch verträgliche Salze. Sie sind natürlichen, synthetischen oder halbsynthetischen Ursprungs.

Regulatorische Peptide, auch als Peptidhormone bezeichnet, sind physiologisch wirksame endogene Peptide. Als Vertreter seien genannt Oxytocin, Vasopressin, Adrenocorticotropes Hormon, Prolactin, Luteinisierendes Hormon-Releasing Hormon (LH-RH), Insulin, Glucagon, Gastrin, Sekretin und Somatostatin.

Von besonderer therapeutischer Bedeutung sind z.B. Insulin sowie insbesondere die LH-RH-Analoga, wie z.B. das Buserelinacetat, die bei kontinuierlicher Verabreichung durch Blockade der Hypophyse zu einer stark verringerten Ausschüttung der gonadotropen Hormone führen und damit zu einer Hemmung der Sekretion von Östradiol und Testosteron. Diese Wirkungen lassen sich zur Behandlung des Mammacarcinoms, der Endometriose, des Prostatacarcinoms und der Pubertas praecox nutzen.

Zur Mikroverkapselung der Peptide wird erfindungsgemäß Poly-D(-)-3-hydroxybuttersäure (PHB) als Trägerstoff, wobei unter Trägerstoff auch das Wandmaterial verstanden wird, eingesetzt. Die PHB wird mikrobiologisch gewonnen (vgl. Pharm. Ind. 45, Seiten 525 - 527 (1983)). Damit entfallen Bedenken wegen eventueller Katalysatorrückstände. Sie wird industriell hergestellt und ist leicht erhältlich. Polymere mit unterschiedlichem Molekulargewicht stehen zur Verfügung. PHB ist biologisch abbaubar. Im Organismus wir sie hydrolytisch und enzymatisch zu dem Monomer 3-Hydroxybuttersäure abgebaut. Diese stellt eine physiologische Substanz dar.

Das Verfahren zur Herstellung der Mikrokapseln ist dadurch gekennzeichnet, daß man

a) eine wäßrige Lösung des Peptids in einer Lösung der PHB in einem halogenierten, aliphatischen $C_1$-$C_4$-Kohlenwasserstoff emulgiert, zu dieser Emulsion zur Phasentrennung Silikonöl zugibt, anschließend mit einem gesättigten flüssigen Kohlenwasserstoff versetzt und die entstandenen Mikrokapseln abtrennt, oder

b) eine wäßrige Lösung oder Suspension des Peptids in einer Lösung der PHB in einem halogenierten, aliphatischen $C_1$-$C_4$-Kohlenwasserstoff emulgiert, die erhaltene Emulsion in der wäßrigen Lösung eines Tensids oder Kolloids emulgiert, das organische Lösungsmittel

- 4 -

0172422

verdampft und die entstandenen Mikrokapseln abtrennt, oder

c) das Peptid in einer Lösung der PHB in einem halogenierten, aliphatischen $C_1$-$C_4$-Kohlenwasserstoff suspendiert, diese Suspension in der wäßrigen Lösung eines Tensids oder Kolloids emulgiert, das organische Lösungsmittel verdampft und die entstandenen Mikrokapseln abtrennt.

Die Abtrennung der Mikrokapseln erfolgt zweckmäßig durch Filtration. Anschließend werden die gemäß Verfahren a) erhaltenen Kapseln vorzugsweise mit dem gesättigten Kohlenwasserstoff gewaschen und die gemäß Verfahren b) bzw. c) erhaltenen Kapseln wäscht man beispielsweise mit Wasser.

Als halogenierte, aliphatische $C_1$-$C_4$-Kohlenwasserstoffe eignen sich insbesondere Chloroform und Methylenchlorid.

Die Lösungen, Emulsionen und/oder Suspensionen werden unter Rühren zusammengegeben. Es ist von Vorteil, das Rühren über einen gewissen Zeitraum fortzusetzen. Die jeweiligen Verfahrensschritte werden zweckmäßigerweise bei Temperaturen zwischen etwa 0° und 50°C, vorzugsweise bei Raumtemperatur, durchgeführt. Die Größe der Mikrokapseln ist für Injektionszubereitungen vorzugsweise nicht größer als 200 µm.

Bei Verfahren a) liegen in der Emulsion Tropfen vor, die nach Zugabe von Silikonöl von einer PHB-Hülle umgeben sind. Diese relativ weiche Polymerhülle wird durch Zusatz des Kohlenwasserstoffs, vorzugsweise durch Zusatz von Petrolether, Heptan oder Pentan, gehärtet.

Bei Verfahren b) wird mit einer doppelten Emulsion gearbeitet. Zunächst entsteht eine Wasser-in-Öl-Emulsion, die in die wäßrige Lösung des Tensids oder Kolloids eingerührt wird, wobei dann eine (W/O)W-Emulsion entsteht.

Es bilden sich kleine Tröpfchen der Primäremulsion in dem Verkapselungsmedium. Unter Rühren verdampft allmählich das organische Lösungsmittel, so daß sich die Tröpfchen verfestigen und Mikrokapseln bilden.

Vorzugsweise wird bei Verfahren b) eine wäßrige Lösung des Peptids eingesetzt. Das Verfahren wird z.B. wie folgt durchgeführt.

Während das Peptid in Wasser gelöst wird, wird die PHB in Methylenchlorid, Chloroform oder einem Gemisch aus beiden gelöst. Das Verhältnis zwischen wäßriger Lösung und organischer Lösung beträgt 1 : 2 bis 1 : 10, bevorzugt ist ein solches von ca. 1 : 5. Zur Emulsion wird unter Rühren die wäßrige Lösung in die organische Phase eingegossen.
Zur Aufnahme dieser Primäremulsion dient eine wäßrige Lösung von Tensiden oder Hydrokolloiden. Als Tenside kommen z.B. Polyvinylalkohol, Na-laurylsulfat oder Dioctylsulfosuccinat-Na in Frage, als Hydrokolloide, z.B. Gelatine, Hydroxypropylcellulose oder Polyvinylpyrrolidon. Bevorzugt werden Polyvinylalkohol in einer Konzentration von 0,5 - 5 Gewichts-%, Gelatine in einer Konzentration von 1 - 4 Gewichts-% eingesetzt. Diese wäßrigen Lösungen dienen als Mikroverkapselungsmedium. Sie werden in einer Menge verwendet, die der fünffachen bis zehnfachen Menge der Primäremulsion entspricht.

Ebenfalls unter Rühren wird nun die Primäremulsion in die Tensid- bzw. Kolloidlösung eingegossen. Das Rühren wird so lange fortgesetzt, bis das organische Lösungsmittel, also Chloroform, Methylenchlorid oder ihre Mischung, verdampft ist. Dies kann bei nahezu 0°C, bei Raumtemperatur oder bei erhöhter Temperatur von 30° oder 40°C erfolgen. Die organischen Lösungsmittel können auch durch Anlegen eines leichten Vakuums verdampft werden.

0172422

- 6 -

Wichtig ist, daß das Entfernen der organischen Phase nicht zu schnell geschieht, damit man gleichmäßig geformte, intakte Mikrokapseln ohne Löcher oder andere Fehlstellen erhält.

Nachdem das organische Lösungsmittel vollständig entfernt ist, läßt man die Mikrokapseln absetzen, dekantiert das überstehende Medium ab, isoliert die Mikrokapseln durch Filtration, wäscht sie mit Wasser und trocknet sie. Nach dem Trocknen erhält man ein freifließendes Pulver aus feinen Kügelchen.

Gemäß Verfahren c) wird das Peptid z.B. in einer Lösung der PHB in Chloroform, Methylenchlorid oder einem Gemisch aus beiden suspendiert. Der Lösung kann auch ein geringer Anteil an Aceton oder einem Alkohol wie Methanol oder Ethanol bis maximal 10 % zugesetzt sein.
Als Mikroverkapselungsmedium dient ebenfalls eine Tensid- oder Hydrokolloidlösung. Als Tenside kommen z.B. Polyvinylalkohol, Natriumlaurylsulfat oder Natrium-Dioctylsulfosuccinat in Betracht, als Hydrokolloide z.B. Gelatine, Hydroxypropylcellulose oder Polyvinylpyrrolidon. Bevorzugt sind Polyvinylalkohol in einer Konzentration von 0,2 - 5 Gewichts-% und Gelatine in einer Konzentration von 1 - 4 Gewichts-%.
Das Verhältnis von organischer zu wäßriger Phase beträgt 1 : 5 bis 1 : 50, vorzugsweise 1 : 10 bis 1 : 25.

Unter Rühren wird die Suspension des Peptids in die Tensid- bzw. Kolloidlösung gegossen. Es bildet sich eine Emulsion. Sie wird solange gerührt, bis das organische Lösungsmittel verdampft ist. Dies kann bei nahezu 0°C, bei Raumtemperatur oder bei höherer Temperatur wie 30°C oder 40°C erfolgen. Es ist auch möglich, die Temperatur im Laufe des Mikroverkapselungsprozesses allmählich zu steigern. Weiterhin ist es möglich, durch Anlegen eines leichten Vakuums das Entfernen des organischen Lösungsmittels zu beschleunigen. Wichtig ist, daß die Verdunstung allmählich vor sich geht, damit man einwandfreie Mikrokapseln erhält. Beim Verdunsten des organischen Lösungsmittels verfestigen sich die Emulsionströpfchen. Es entstehen Mikrokapseln.

Sobald das organische Lösungsmittel verdampft ist, werden die Mikrokapseln abfiltriert, mit Wasser gewaschen und getrocknet. Man erhält ein freifließendes Pulver aus Mikrokügelchen.

Üblicherweise fallen bei den geschilderten Verfahren die Mikrokapseln in unterschiedlichen Korngrößen an. Will man für besondere Zwecke ein bestimmte Teilchengröße nicht überschreiten bzw. unterschreiten, so lassen sie sich durch Siebe passieren und so fraktionieren.

Es ist aber auch möglich, durch Variieren der Herstellungsbedingungen die Kapselgröße zu beeinflussen. Insbesondere läßt sich die Teilchengröße durch die Viskosität der Polymerlösung, die Menge an Tensid oder Kolloid in der wäßrigen Phase und die Umdrehungsgeschwindigkeit beim Rühren steuern. Im allgemeinen nimmt die Teilchengröße mit abnehmender Viskosität der Polymerlösung, zunehmendem Anteil an Tensid oder Kolloid und mit zunehmender Umdrehungsgeschwindigkeit ab.

Weiterhin lassen sich die Eigenschaften der Mikrokapseln dadurch beeinflussen, daß man ihren Beladungsgrad ändert, d.h. das Verhältnis von Wirkstoff zu Polymer unterschiedlich wählt. Es kann 1 : 3 bis 1 : 100 betragen; bevorzugt ist das Verhältnis 1 : 50 bis 1 : 20.

Die erfindungsgemäßen Verfahren erlauben es, injizierbare Mikrokapseln aus PHB zur kontrollierten Freisetzung von Peptiden herzustellen.

Die erfindungsgemäßen Mikrokapseln können die Peptide über verschieden lange Zeiträume entsprechend den therapeutischen Anforderungen gleichmäßig freisetzen. Diese Zeiträume können Wochen bis Monate betragen.

Da PHB biologisch abbaubar ist, können die Mikrokapseln nach Suspension in einem geeigneten Medium injiziert werden und erleichtern somit im Vergleich zur Applikation von Implantaten die Anwendung.

Die Erfindung betrifft daher auch Injektionszubereitungen enthaltend die erfindungsgemäßen Mikrokapseln. Letztere werden in einem physiologisch verträglichen Medium suspendiert. Als solche kommen z.B. wäßrige 1 - 2 %ige (Gewichts-%) Lösungen von Polyvinylpyrrolidon, Natrium-Carboxymethylcellulose oder Polyethylenglykolsorbitan-monostearat in Betracht. Das wäßrige Medium kann auch physiologische Kochsalzlösung enthalten.

Zur Verdeutlichung der Ausführungen sind nachfolgend Beispiele ausgeführt. Die geschilderten Herstellungsvarianten sollen keineswegs den Erfindungsgegenstand einschränken.

## Beispiel 1

900 mg PHB wurden in 100 ml Chloroform gelöst. Unter Rühren mit 750 Upm wurde eine Lösung von 100 mg Buserelinacetat in 2 ml Wasser auf einmal zugegeben. Zu der entstandenen Emulsion wurden innerhalb von 30 Minuten 40 ml niedrig viskoses Silikonöl (z.B. Silikonöl AK 10) zugetropft. Es wurde weitere 15 Minuten lang gerührt. Anschließend wurden 100 ml Petrolether (Sp. 60° - 70°C) während 30 Minuten zugetropft. Es wurde weitere 30 Minuten gerührt. Dann wurden die Mikrokapseln abfiltriert, mit Petrolether nachgewaschen und getrocknet.

Ausbeute:        500 mg
Teilchengröße: 40 - 120 μm

Beispiel 2

50 mg Kristallinsulin wurden in 5 ml verdünnter Salzsäure, 650 mg PHB in 25 ml Chloroform gelöst. Die beiden Lösungen wurden zusammen gegeben und unter Rühren bei 500 Upm emulgiert. Die entstandene Emulsion wurde in 500 ml einer 4 %igen (Gewichts-%), wäßrigen Polyvinylalkohollösung gegossen. Dabei wurde mit 700 Upm gerührt. Das Rühren wurde 3 Stunden fortgesetzt. Danach wurden die gebildeten Mikrokapseln abfiltriert, mit Wasser gewaschen und getrocknet.

Ausbeute:       370 mg
Teilchengröße: 50 - 250 µm

Beispiel 3

1900 mg PHB wurden in 20 ml Chloroform gelöst. In der Lösung wurden 100 mg Kristallinsulin suspendiert. Unter Rühren bei 500 Upm wurde die Suspension in 500 ml einer 4 %igen (Gewichts.-%), wäßrigen, 25°C warmen Gelatinelösung gegossen. Es wurde 5,5 Stunden gerührt. Die entstandenen Mikrokapseln wurde abfiltriert, mit warmen Wasser gewaschen und getrocknet.

Ausbeute:       1468 mg
Teilchengröße: 40 - 150 µm

Beispiel 4

500 mg PHB wurden in 20 ml Chloroform gelöst. In der Lösung wurden 100 mg Buserelinacetat suspendiert. Unter Rühren bei 800 Upm wurde die Suspension in 500 ml einer 4 %igen (Gewichts-%), wäßrigen 30°C warmen Gelatinelösung gegossen. Es wurde noch 2 Minuten lang bei 800 Upm, anschließend 4,5 Stunden bei 400 Upm gerührt. Die entstandenen Mikrokapseln wurden abfiltriert, mit warmem
Wasser gewaschen und getrocknet.

Ausbeute:       413 mg
Teilchengröße: 20 - 150 µm

Patentansprüche:

1. Mikrokapseln von regulatorischen Peptiden mit kontrollierter Wirkstoff-Freisetzung, dadurch gekennzeichnet, daß sie Poly-D(-)-3-hydroxybuttersäure als biologisch abbaubaren Trägerstoff enthalten.

2. Mikrokapseln gemäß Anspruch 1, dadurch gekennzeichnet, daß sie Buserelinacetat enthalten.

3. Verfahren zur Herstellung von Mikrokapseln gemäß Anspruch 1, dadurch gekennzeichnet, daß man

a) eine wäßrige Lösung des Peptids in einer Lösung der Poly-D(-)-3-hydroxybuttersäure in einem halogenierten, aliphatischen $C_1-C_4$-Kohlenwasserstoff emulgiert, zu dieser Emulsion zur Phasentrennung Silikonöl zugibt, anschließend mit einem gesättigten flüssigen Kohlenwasserstoff versetzt und die entstandenen Mikrokapseln abtrennt, oder

b) eine wäßrige Lösung oder Suspension des Peptids in einer Lösung der Poly-D(-)-3-hydroxybuttersäure in einem halogenierten, aliphatischen $C_1-C_4$-Kohlenwasserstoff emulgiert, die erhaltene Emulsion in der wäßrigen Lösung eines Tensids oder Kolloids emulgiert, das organische Lösungsmittel verdampft und die entstandenen Mikrokapseln abtrennt, oder

c) das Peptid in einer Lösung der Poly-D(-)-3-hydroxybuttersäure in einem halogenierten, aliphatischen $C_1-C_4$-Kohlenwasserstoff suspendiert, diese Suspension in der wäßrigen Lösung eines Tensids oder Kolloids emulgiert, das organische Lösungsmittel verdampft und die entstandenen Mikrokapseln abtrennt.

4. Verfahren gemäß Anspruch 3, dadurch gekennzeichnet,
   daß man als halogenierten, aliphatischen $C_1$-$C_4$-Kohlen-
   wasserstoff Chloroform oder Methylenchlorid verwendet.

5. Injektionszubereitung, dadurch gekennzeichnet, daß
   sie Mikrokapseln gemäß Anspruch 1 suspendiert in
   einem physiologisch verträglichen Medium enthält.

Patentansprüche für Österreich:

1. Verfahren zur Herstellung von Mikrokapseln mit kontrollierter Wirkstoff-Freisetzung enthaltend regulatorische Peptide und einen biologisch abbaubaren Trägerstoff, dadurch gekennzeichnet, daß man

a) eine wäßrige Lösung des Peptids in einer Lösung von Poly-D(-)-3-hydroxybuttersäure in einem halogenierten, aliphatischen $C_1$-$C_4$-Kohlenwasserstoff emulgiert, zu dieser Emulsion zur Phasentrennung Silikonöl zugibt, anschließend mit einem gesättigten flüssigen Kohlenwasserstoff versetzt und die entstandenen Mikrokapseln abtrennt, oder

b) eine wäßrige Lösung oder Suspension des Peptids in einer Lösung von Poly-D(-)-3-hydroxybuttersäure in einem halogenierten, aliphatischen $C_1$-$C_4$-Kohlenwasserstoff emulgiert, die erhaltene Emulsion in der wäßrigen Lösung eines Tensids oder Kolloids emulgiert, das organische Lösungsmittel verdampft und die entstandenen Mikrokapseln abtrennt, oder

c) das Peptid in einer Lösung von Poly-D(-)-3-hydroxybuttersäure in einem halogenierten, aliphatischen $C_1$-$C_4$-Kohlenwasserstoff suspendiert, diese Suspension in der wäßrigen Lösung eines Tensids oder Kolloids emulgiert, das organische Lösungsmittel verdampft und die entstandenen Mikrokapseln abtrennt.

2. Verfahren gemäß Anspruch 1, dadurch gekennzeichnet, daß man Buserelinacetat verwendet.

3. Verfahren gemäß Anspruch 1, dadurch gekennzeichnet, daß man als halogenierten, aliphatischen $C_1$-$C_4$-Kohlenwasserstoff Chloroform oder Methylenchlorid verwendet.

4. Verfahren zur Herstellung einer Injektionszubereitung, dadurch gekennzeichnet, daß man Mikrokapseln erhältlich nach Anspruch 1 in einem physiologisch verträglichen Medium suspendiert.